# EUROPÄISCHE PATENTSCHRIFT

(11) **EP 1 279 385 B1**
(45) Veröffentlichungstag und Bekanntmachung des Hinweises auf die Patenterteilung: **21.05.2008**
(21) Anmeldenummer: 02015946.3
(22) Anmeldetag: 17.07.2002
(51) Int. Cl.: A61F 9/008, A61B 5/00

(54) **Nichtinvasive Bestimmung der Temperatur an mit Strahlung behandeltem biologischem Gewebe**
Non-invasive temperature determination for irradiated biological tissue
Détermination non-invasive de la température des tissus vivants irradiés

(30) Priorität: 24.07.2001 DE 10135944
(43) Veröffentlichungstag der Anmeldung: 29.01.2003
(73) Patentinhaber: Medizinisches Laserzentrum Lübeck GmbH, D-23562 Lübeck (DE)
(72) Erfinder: Schüle, Georg, 23564 Lübeck (DE); Brinkmann, Ralf, 23562 Lübeck (DE)
(74) Vertreter: Nöth, Heinz

(56) Entgegenhaltungen:
- WO-A-00/24315
- DE-A- 19 916 653
- DE-A- 19 932 477
- OBERHEIDE: "optoacoustic online control" PROC SPIE, Bd. 4256, Januar 2001 (2001-01), XP001105011
- SCHÜLE: "optoacoustic measurements" PROC SPIE, Bd. 3914, 2000, Seiten 230-236, XP001105005

## Beschreibung

Die Erfindung betrifft eine Vorrichtung zur Behandlung von biologischem Gewebe nach dem Oberbegriff des Patentanspruches 1 oder 2.

Es ist bekannt, mit Hilfe optoakustischer Techniken die Gewebetemperatur bei der thermischen Behandlung des biologischen Gewebes zu ermitteln (K.V. Larin, I.V. Larina, M. Motamedi, R.O. Esenaliev: Monotoring temperature distribution with optoacustic technique in real time. SPIE Proc. 3916: 311-321, 2000).

Aus WO 00/24315 A ist eine Vorrichtung zur Behandlung von biologischem Gewebe mit einer Laserstrahlquelle zur Erzeugung einer gepulsten Behandlungsstrahlung bekannt. Während einer vorgegebenen Behandlungszeit wird mit Hilfe der gepulsten Behandlungsstrahlung, welche über eine Bestrahlungsoptik auf das biologische Gewebe gerichtet wird, eine Laserkoagulation des Gewebes durchgeführt. Mit Hilfe einer zusätzlichen Laserstrahlquelle, welche während der Behandlungszeit zusätzliche Strahlungspulse liefert, werden Expansionen und/oder Kontraktionen des Gewebes erzeugt, welche mittels eines Detektors gemessen werden. Bekannt ist ferner, dass die dabei erzeugten opto-akustischen Signale in ihren Amplituden temperaturabhängig sind.

Aus DE 199 16 653 A ist eine Vorrichtung zur opto-akustischen Gewebedifferenzierung bekannt, bei welcher Laserpulse zur Photokoagulation in das Zielgewebe gerichtet werden. Ferner werden im Zielgewebe Drucktransienten als Messgröße der optischen Eigenschaften des Zielgewebes herangezogen. Die Generierung der Drucktransienten erfolgt mittels kurzer Diagnoseimpulse mit niedriger Energie, welche simultan zur Behandlungsstrahlung in das Zielgewebe geschickt werden. Dabei können die Diagnoseimpulse mittels eines Modulators auf die zur Koagulation eingebrachten längeren Laserpulse aufmoduliert werden.

Aus der DE 199 32 477 A1 ist es bekannt, an biologischem Gewebe durch gepulste Bestrahlung hervorgerufene Materialänderungen mit optoakustischen Techniken zu bestimmen.

Aus G. Schüle, G. Hüttmann, J. Roider, C. Wirbelauer, R. Birngruber, R. Brinkmann: Optacustic measurement during µsirradiation of the retinal pigment epithelium, SPIE, Proc. Vol. 3914: 230236, 2000 ist es bekannt, bei der selektiven Mikrophotokoagulation am Augenhintergrund zur Behandlung von Netzhauterkrankungen mittels µs-Laserpulsen die Temperaturen am behandelten Augenhintergrund zu messen. Die vom ersten Behandlungspuls bewirkte Druckamplitude wurde zur Normierung der Druckamplitude auf die Temperatur des Augenhintergrundes verwendet. Aus der Druckamplitudenerhöhung der nachfolgenden Behandlungsimpulse wurden mit Hilfe der aus Eichmessungen bekannten Abhängigkeit der Temperatur von den Druckamplituden die Temperaturerhöhung und ferner die jeweiligen Absoluttemperaturen bestimmt.

In vielen Bereichen der Augenheilkunde werden unterschiedliche Energiequellen, insbesondere Laser, zur Diagnostik und Behandlung eingesetzt. Dabei wird in aller Regel die gesamte eingestrahlte Energie vom biologischen Gewebe absorbiert und in Wärme umgewandelt, wobei durch die daraus resultierende Temperaturerhöhung der gewünschte Behandlungseffekt erreicht wird. Beispielsweise bei der Laserphotokoagulation wird die Retina des Auges gezielt thermisch koaguliert. Bei den üblichen Bestrahlungen mit Bestrahlungszeiten um 100 ms entstehen Temperaturen über 60°C. Auch bei der Transpupilären Thermotherapie (TTT) werden Temperaturerhöhungen ausgenützt, um einen Gefäßverschluss zu erreichen. Bei der Photodynamischen Therapie (PDT) wird ein vorher injizierter Farbstoff durch Laserbestrahlung am Augenhintergrund aktiviert. Der Wirkstoff entfaltet nur an den Zellen seine Wirkung, an denen er gebunden ist. Hierbei wird ebenfalls fast die gesamte eingestrahlte Energie im Farbstoff und in der Retina absorbiert und in Wärme umgewandelt. Während der jeweiligen Bestrahlungszeit (Pulsdauer) mit relativ langen Behandlungs- und Strahlungspulsen in der Größenordnung von µs bis mehreren hundert Sekunden kann es zu einer Temperaturerhöhung des behandelten biologischen Gewebes, insbesondere des Augenhintergrundes kommen, die zu einer nicht beabsichtigen Schädigung von Netzhautbereichen führt. Eine nichtinvasive Real-Time-Temperaturbestimmung kann bei derartigen Augenbehandlungen bislang noch nicht durchgeführt werden.

Aufgabe der Erfindung ist es daher, eine Vorrichtung zur Behandlung von biologischem Gewebe der eingangs genannten Art zu schaffen, bei der die Temperaturänderung während eines jeweiligen Laserpulses der Behandlungsstrahlung erfasst werden kann.

Diese Aufgabe wird erfindungsgemäß durch die kennzeichnenden Merkmale des Patentanspruches 1 oder 2 gelöst.

Bei der Erfindung werden während der jeweiligenBestrahlungszeit der Behandlungsstrahlung zusätzliche Strahlungpulse mit geringerer Pulsdauer und geringerer Energie als bei der Behandlungsstrahlung auf das behandelte biologische Gewebe gerichtet oder es wird die Behandlungsstrahlung kurzzeitig abgeschaltet und wieder eingeschaltet. Die zusätzlichen Strahlungspulse oder die kurzzeitigen Abschaltungen der Behandlungsstrahlung können im wesentlichen in gleichen Zeitabständen erfolgen. Die bei Anwendung der zusätzlichen Strahlungsimpulse entstehenden thermischen Gewebeexpansionen und die bei den kurzzeitigen Abschaltungen der Behandlungsstrahlung entstehenden Gewebekontraktionen werden durch Druckmessung oder durch optische Messung erfasst. Aus den jeweiligen Messsignalen, welche von den zusätzlichen Strahlungspulsen (Messstrahlungspulsen) oder den kurzzeitigen Abschaltungen der Behandlungsstrahlung veranlasst werden, wird die Temperaturerhöhung bestimmt, insbesondere werden die jeweiligen Absolutwerte der Temperatur bestimmt.

Bei der Erfindung ist die Pulsenergie der zusätzlich eingestrahlten Strahlungspulse bzw. die Abschaltzeit, während welcher die Behandlungsstrahlung mehrmals abgeschaltet wird, konstant. Die in das Gewebe applizierte Energie bewirkt eine temperaturabhängige, optoakustisch auswertbare thermische Expansion des Gewebes, für die ein Maß der Grüneisenkoeffizient ist. Durch Eichmessungen gewinnt man eine Eichkurve, welche einen linearen Anstieg der akustischen Amplituden mit der Temperatur gemäß dem Grüneisenkoeffizient aufweist (G. Schüle, G. Hüttmann, J. Roider, C. Wirbelauer, R. Birngruber, R. Brinkmann: Optoacustic measurement during µsirradiation of the retinal pigment epithelium, SPIE, Proc., Vol. 3914: 230-236, 2000).

Vorzugsweise kommen bei der Erfindung kurzzeitige Laserpulse zum Einsatz mit wenigen ns, beispielsweise 8 ns und geringer Pulsenergie mit wenigen µJ, beispielsweise 5 µJ zum Einsatz. Die Laserpulse können auch länger sein. Aufgrund der hohen Lichtabsorption am Augenhintergrund, insbesondere des retinalen Pigmentepitels (RPE) genügen die geringen Pulsenergien, um ausreichende Messwerte, insbesondere der Druckmessung (akustische Messung) und der optischen Messung, zu erhalten. Bei der Druckmessung wird vorzugsweise ein Maximalwert oder ein Integral der jeweils gemessenen Druckhalbwelle für die Auswertung bei der Temperaturbestimmung verwendet. Es können jedoch auch andere Algorithmen, beispielsweise die Steigung oder eine Fourier-Transformation der Messsignale bei der Auswertung verwendet werden.

Als optische Messung wird bevorzugt eine Interferenzmessung mit Hilfe eines Interferometers, insbesondere Faserinterferometers, dessen Messstrahl in die Behandlungsoptik der Behandlungsstrahlung eingekoppelt wird, verwendet.

Vor dem Einschalten der Behandlungsstrahlung wird durch einen zusätzlichen kurzzeitigen Strahlungspuls oder unmittelbar nach Einschalten der Behandlungsstrahlung durch kurzzeitiges Ausschalten aus der dabei entstehenden Gewebeexpansion bzw. -kontraktion ein Messsignal gewonnen, das auf die normale Körpertemperatur, beispielsweise die menschliche Körpertemperatur von 37°C normiert wird.

Aufgrund der ständigen Überwachung der Temperatur auch während der jeweiligen Bestrahlungszeit (Pulsdauer) der Behandlungsstrahlung ist eine Steuerung der Behandlungsstrahlung in Abhängigkeit von der jeweils ermittelten Temperatur am Bestrahlungsort möglich. Dabei kann insbesondere die Pulsenergie oder Pulsleistung des Behandlungsstrahles gesteuert werden, um die gewünschte Temperatur am Bestrahlungsort zu erhalten.

### [Beispiele]

Anhand der Figuren wird die Erfindung noch näher erläutert.

Es zeigt
- Fig. 1: ein erstes Ausführungsbeispiel für eine erfindungsgemässe Vorrichtung;
- Fig. 2: ein zweites Ausführungsbeispiel; und
- Fig. 3: ein drittes Ausführungsbeispiel;
- Fig. 4 bis 12: Ausführungsbeispiele für Detektoranordnungen, mit denen die thermische Gewebeexpansion bzw. -kontraktion erfasst werden kann; und
- Fig. 13: elektrische Signale, welche optoakustische Transienten darstellen.

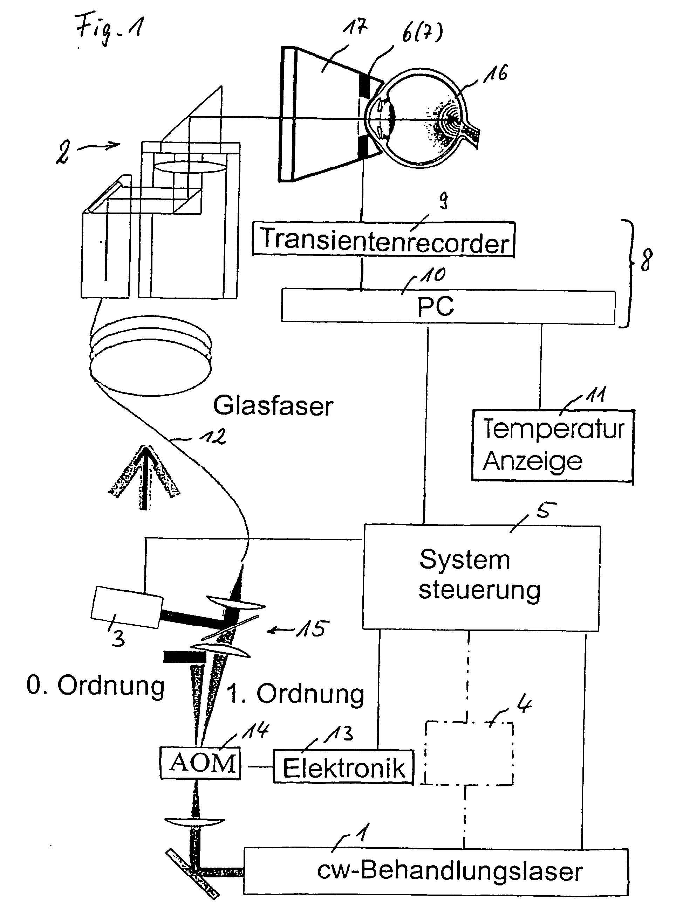

Die in den Figuren 1 bis 3 teilweise mit Blockschaltbild dargestellte Vorrichtung beinhaltet eine Behandlungsstrahlquelle, beispielsweise eine Laserstrahlquelle, insbesondere einen cw-Laser. Mit der Behandlungsstrahlquelle 1 wird die für die Behandlung des Augenhintergrundes verwendete Behandlungsstrahlung erzeugt. Die Behandlungsstrahlung wird aufbereitet und über einen von einer zugeordneten Steuerelektronik 13 gesteuerten Schalter 14, z. B., akustooptischen Modulator, mechanischen Shutter oder dergleichen, und eine Koppleroptik 15 in einen Lichtleiter, z. B. Glasfaser 12 eingekoppelt. Am Glasfaserende befindet sich eine Bestrahlungsoptik 2, beispielsweise in Form einer Spaltlampe, mit der die Behandlungsstrahlung auf den Fundus, insbesondere die Retina eines Auges 16 gerichtet wird.

Die Behandlungsstrahlquelle 1 wird von einer Steuereinrichtung 5 gesteuert. Durch die Steuereinrichtung 5 kann insbesondere die Strahlungsenergie und die Zeitdauer, mit welcher die Behandlungsstrahlung auf den Augenhintergrund gerichtet wird, gesteuert werden.

Die in den Fig. 1 bis 3 dargestellten Vorrichtungen besitzen ferner eine zusätzliche Strahlquelle 3 in Form eines gepulsten Lasers. Die Strahlung der zusätzlichen Strahlquelle 3 kann mit Hilfe der Koppleroptik 15, welche hierzu einen halbdurchlässigen Spiegel aufweisen kann, in den Strahlengang der Behandlungsstrahlung eingekoppelt werden. Bei den dargestellten Ausführungsbeispielen erfolgt diese Einkoppelung vor oder gleichzeitig mit dem Einkoppeln der Behandlungsstrahlung in den Lichtleiter 12. Die dargestellten Vorrichtungen kommen bevorzugt bei der Phototherapie bestimmter Stellen am Augenhintergrund, insbesondere pigmentierten Gewebes, beispielsweise der Photodynamischen Therapie (PDT) zum Einsatz. Dabei wird ein vorher injizierte Farbstoff durch Laserbestrahlung am Augenhintergrund aktiviert. Es werden hierzu beispielsweise Laserpulse mit einer Bestrahlungszeit (Pulsdauer) von ca. 90 sec zum Einsatz gebracht. Diese Pulsdauer entspricht in aller Regel bei der Photodynamischen Therapie auch der Behandlungszeit.

Ferner können die dargestellten Vorrichtungen zur Photokoagulation mit Laserstrahlung als Behandlungsstrahlung zum Einsatz gebracht werden. Die Behandlungsdauer beträgt ca. 100 ms. Es wird hierzu ein cw-Laserpuls mit entsprechender Pulsdauer zur Anwendung gebracht. Ferner kann eine Transpupilläre Thermotherapie (TTT) durchgeführt werden. Hierzu wird im Sekundenbereich bestrahlt und durch die dabei erzielte Temperaturerhöhung wird ein Gefäßverschluss erreicht.

Außerdem kann eine selektive RPE-Behandlung durchgeführt werden. Hierbei kommen Laserpulse als Behandlungsstrahlung zum Einsatz mit einer Pulsdauer von ca. 30 µs während einer Behandlungszeit von ca. 300 ms.

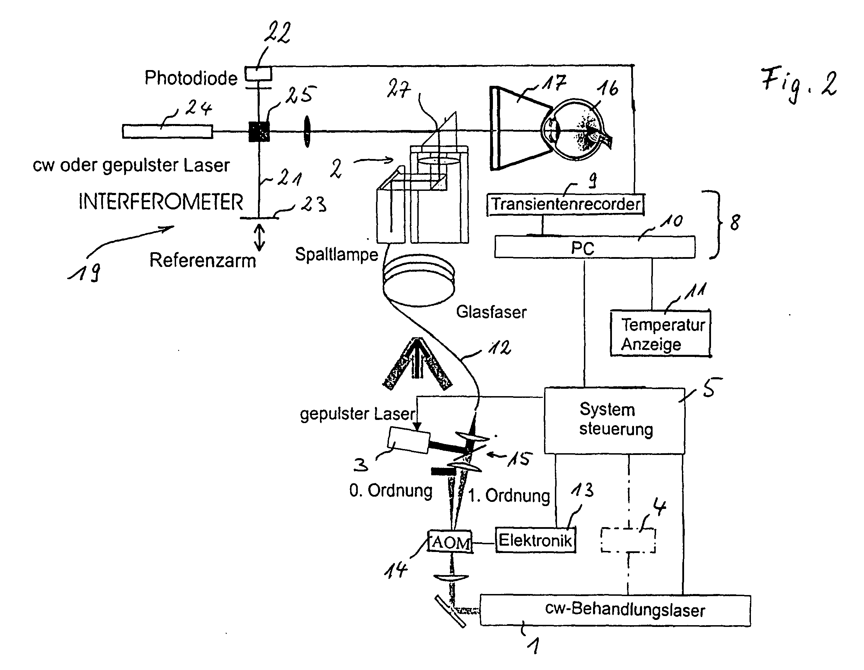

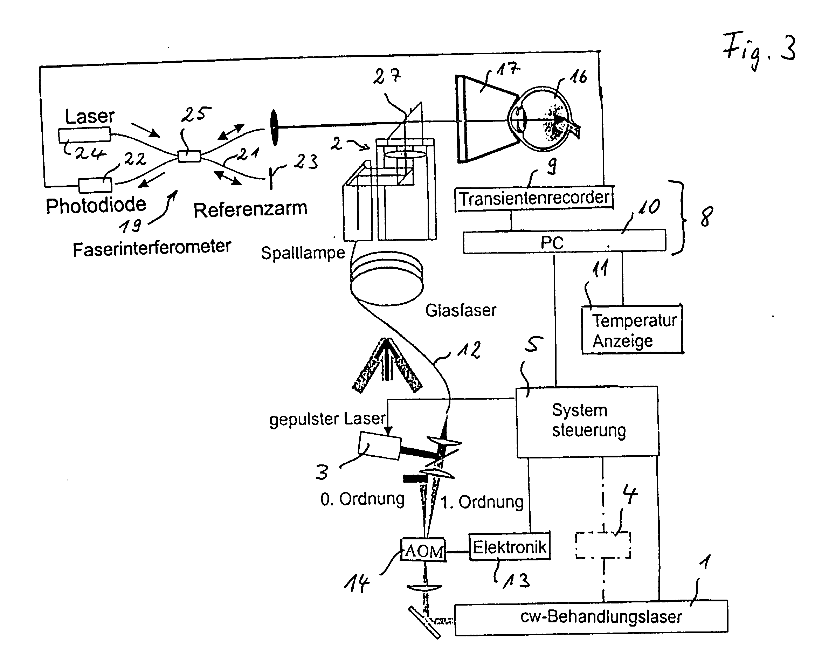

Mit den dargestellten Vorrichtungen werden die Strahlungspulse der Behandlungsstrahlung mit zusätzlichen kurzzeitigen Strahlungspulsen überlagert. Die Pulsdauer der zusätzlichen Strahlungspulse sind um das einige Hundert-bis einige Tausendfache zeitlich kürzer bemessen als die Behandlungszeiten (Pulsdauern) der Behandlungsstrahlung. Die Pulsdauern der zusätzlichen Strahlung, insbesondere der zusätzlichen Laserpulse, welche von der zusätzlichen Strahlquelle 3 erzeugt werden, haben eine Pulsdauer von einigen ns, beispielsweise 5 bis 100 ns. Auch die Pulsenergie ist äußerst gering und beträgt einige µJ, beispielsweise etwa 5 µJ. Die in regelmäßigen zeitlichen Abständen von der Koppleroptik 15 in die Behandlungsstrahlung eingekoppelten zusätzlichen Strahlungspulse besitzen eine konstante Pulsenergie. Durch die zusätzlichen Pulse werden kurzzeitige thermische Expansionen des behandelten biologischen Gewebes veranlasst, die durch Druckmessung (Fig. 1) oder durch Interferenzmessung (Fig. 2 und 3) erfasst werden können. Die Amplituden dieser kurzzeitigen Expansionen sind proportional der Temperatur des biologischen Mediums.

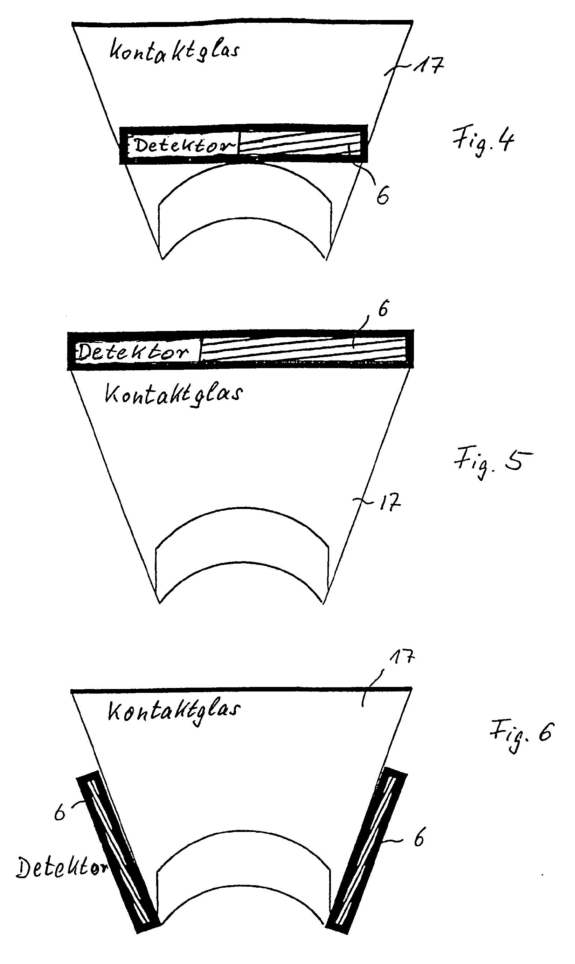

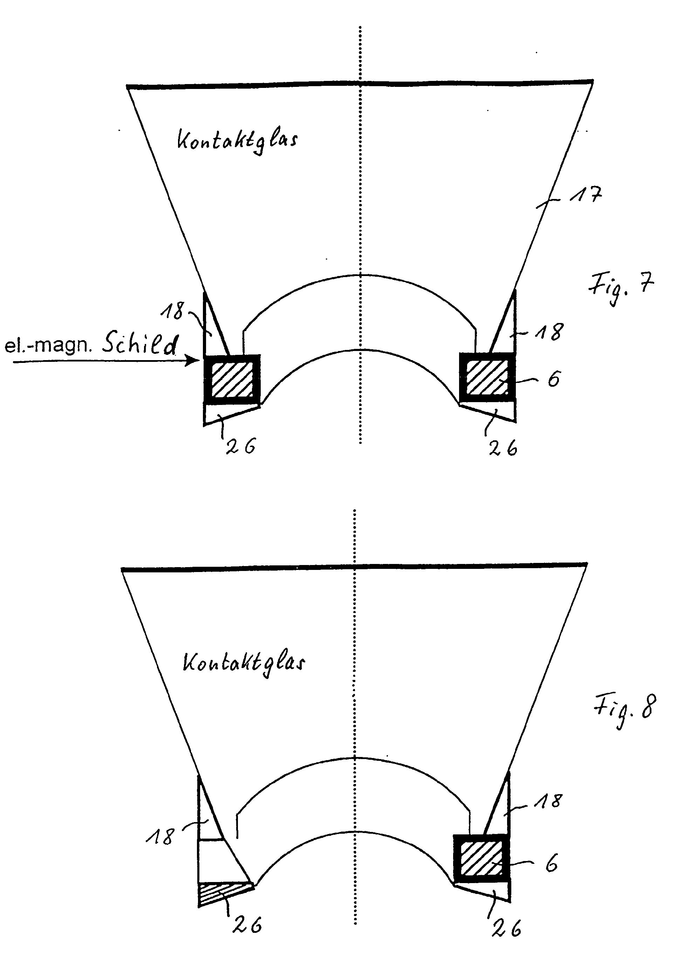

Bei dem in der Fig. 1 dargestellten Ausführungsbeispiel werden diese thermischen Expansionen am Augenhintergrund von einem akustischen Wandler 6, welcher die durch die Gewebeexpansionen ausgesendeten Druckwellen empfängt und in elektrische Signale wandelt, erfasst. Hierzu ist der akustische Wandler 6 in oder an einem Kontaktglas 17, welches auf das Auge aufsetzbar ist und über den Augenbulbus die Druckwellen aufnimmt, angeordnet. Als akustische Wandler 6 können Schalldetektoren mit den in den Fig. 4, 5 und 6 gezeigten Anordnungen vorgesehen sein. Die Schalldetektoren können beispielsweise aus piezoelektrischen Material bestehen. Bei dem in der Fig. 7 dargestellten Ausführungsbeispiel ist ein ringförmiger akustischer Wandler 6 vorgesehen, der ebenfalls aus piezoelektrischen Material bestehen kann. Bei dem in der Fig. 8 dargestellten Ausführungsbeispiel befindet sich der Wandler 6 an einer Seite des Kontaktglases 17.

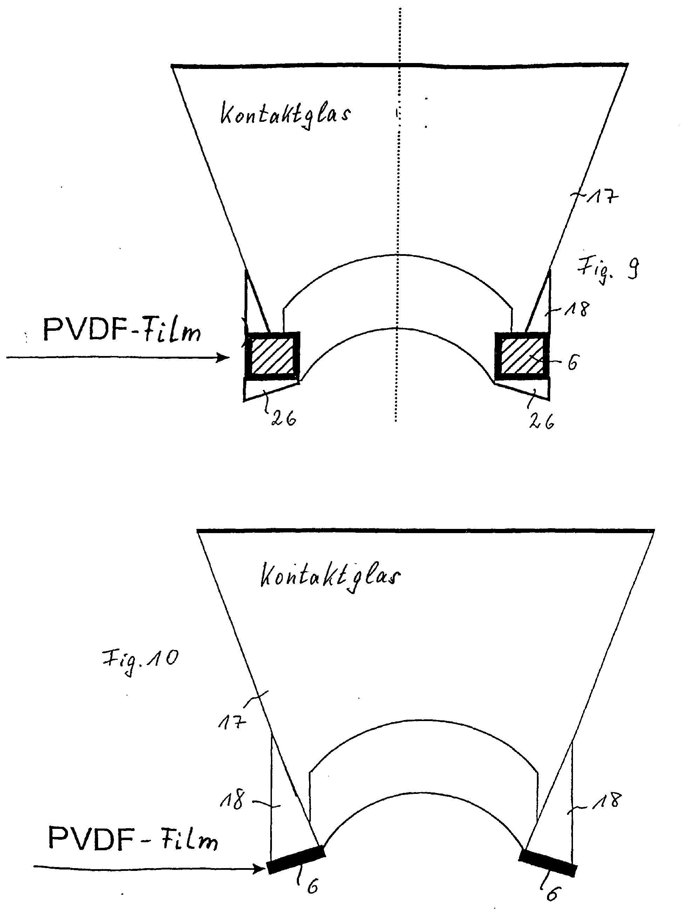

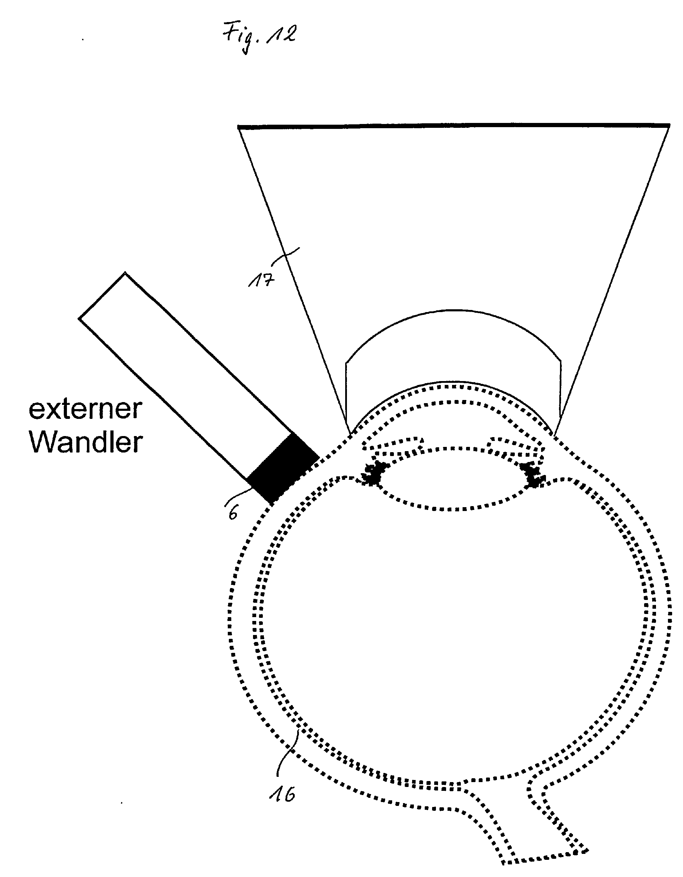

Bei den Ausführungsbeispielen der Fig. 9 und 10 besteht der akustische Wandler 6 aus Polyvinylidenfluorid (PVDF). Bei dem in der Fig. 10 dargestellten Ausführungsbeispiel wird ein PVDF-Film als piezoelektrisches Element vorgesehen. An ihrer Vorderseite besitzen die in den Figuren 7 bis 10 dargestellten akustischen Wandler 6 eine akustische Impedanzanpassung 26 in Form eines konischen, des Auge angepassten Aufsetzrandes des Kontaktglases 17, um eine bessere Schalleinleitung in den Wandler 6 zu erreichen. Durch die abgeschrägte Kontaktfläche wird eine verbesserte Berührung mit der Augenoberfläche gewährleisten. An der Rückseite besitzen der Wandler 6 eine akustisch absorbierende Schicht 18, um Reflektionen der Schallwellen zu verringern. Bei dem in der Figur 12 dargestellten Ausführungsform wird ein externer auf das Auge 16 aufsetzbarer Wandler 6 verwendet.

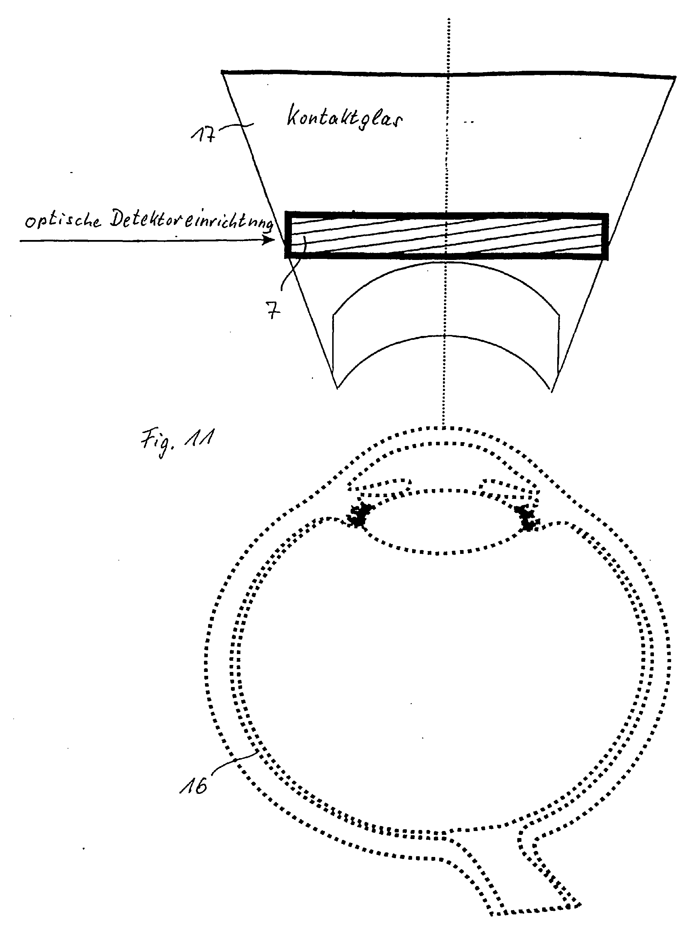

Bei dem in der Fig. 11 dargestellten Ausführungsbeispiel ist in das Kontaktglas 17 ein interferrometrischer Detektor 7 eingebaut. In Abhängigkeit von den durch thermische Expansion erzeugten Druckwellen am Augenhintergrund ändern sich Interferenzerscheinungen am Detektor 7. Diese können dann beispielsweise mit Hilfe einer Photodiode 22, wie in den Ausführungsbeispielen der Fig. 2 und 3, erfasst werden und mit Hilfe eines Transientenrecorders 9 in einem Rechner 10 einer Auswerteeinrichtung 8 im Hinblick auf absolute Temperaturwerte ausgewertet werden.

Auch die Messwerte der Detektoren, welche in den Fig. 3 bis 9 dargestellt sind, werden als elektrische Transienten dem Transientenrecorder 9 in Fig. 1 zugeleitet und in dem Rechner 10 in die Absolutwerte der Temperatur umgerechnet. Die Absolutwerte der Temperatur können von einer Temperaturanzeigeeinrichtung 11 angezeigt werden. Diese ist an den Rechner 10 angeschlossen.

Bei den in den Fig. 2 und 3 dargestellten Ausführungsbeispielen wird zur Erfassung der thermischen Gewebeexpansionen ein Interferrometer 19, welches als zweistrahliges Interferrometer ausgebildet ist, verwendet. Das Interferometer 19 der Figur 3 ist als Faserinterferometer ausgebildet. Das dargestellte Interferrometer 19 besitzt in bekannter Weise eine Strahlquelle 24, welche als cw- oder gepulster Laser ausgebildet sein kann. Die von der Strahlquelle 24 ausgesendete Strahlung wird in einem Messstrahlengang 20, beispielsweise über einen Strahlteiler 27 an der Bestrahlungsoptik 2 in den Behandlungsstrahlengang eingeleitet und trifft am Augenhintergrund auf die vom Behandlungsstrahl behandelte Stelle auf. Ferner besitzt das dargestellte Interferrometer 19 einen Referenzreflektor 23, dem die von der Strahlquelle 24 ausgesendete Strahlung über einen Strahlteiler 25 in einem Referenzstrahlengang 21 zugeleitet wird. Der Referenzreflektor 23 kann zu Instagezwecken in Richtung des Referenzstrahlenganges 21 bewegt werden. Während der Behandlung und Temperaturmessung bleibt der Abstand zum Strahlteiler 25 konstant.

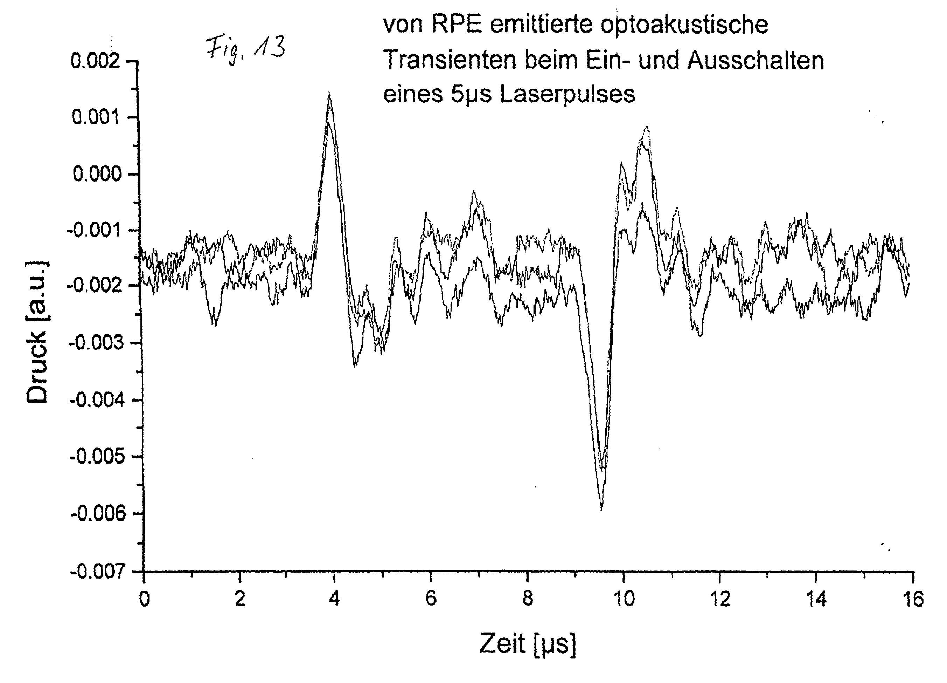

Die vom Augenhintergrund reflektierte Strahlung der Strahlquelle 24 und die vom Referenzreflektor 23 reflektierte Strahlung werden am Strahlteiler 25 zusammengeführt und es entstehen in Abhängigkeit von den thermischen Expansionen am Augenhintergrund Interferenzen, die von einer Photodiode 22 erfasst werden. Die Änderungen bzw. Übergänge der Referenzerscheinungen werden dem Transientenrecorder 9 in Form elektrischer Signale zugeleitet. In der Figur 13 sind optoakustische Transienten dargestellt, welche beim Ein- und Ausschalten eines auf RPE applizierten 5µs Laserpulses gemessen werden. Beim Einschalten ergibt sich eine positive Druckwelle durch die thermische Ausdehnung. Beim Abschalten entsteht durch das Abkühlen eine negative Druckwelle (Zugwelle). Diese Amplitude ist wegen der nun höheren Temperatur größer. Dies entspricht der absoluten Temperaturerhöhung. Im Rechner 10 der Auswerteeinrichtung 8 werden dann hieraus die Temperaturwerte, wie beim Ausführungsbeispiel der Fig. 1 bestimmt. Die Absolutwerte der Temperatur können von der Anzeigeeinrichtung 11 ebenfalls angezeigt werden.

Bei beiden Ausführungsbeispielen der Fig. 1 und 2 werden die Temperaturwerte der Steuereinrichtung 5 zugeleitet. In Abhängigkeit von diesen Temperaturwerten erfolgt die Steuerung bzw. Betätigung der Behandlungsstrahlquelle 1, wie oben schon erläutert wurde.

Die Ausführungsbeispiele der Fig. 1 bis 3 wurden im Zusammenhang mit einer zusätzlichen gepulsten Strahlquelle 3 erläutert. Diese gepulste Strahlquelle 3 kann vorzugsweise in fest vorgegebenen Zeitabständen die kurzzeitigen zusätzlichen Strahlungspulse erzeugen, welche mit Hilfe der Koppleroptik 15 in den Strahlengang der Behandlungsstrahlquelle 1 eingekoppelt werden. Es ist auch möglich, dass die zusätzliche Strahlquelle 3 durch die Steuereinrichtung 5 gesteuert wird.

Als Alternative zur zusätzlichen Strahlungsquelle 3 kann eine Schalteinrichtung 4 vorgesehen sein, welche die Behandlungsstrahlquelle 1 während der jeweiligen Bestrahlungszeit kurzzeitig (einige Nanosekunden) und bei langer Behandlungs-Bestrahlungszeit bis zu einigen Sekunden abschaltet und wieder einschaltet. Die Schalteinrichtung 4 kann hierzu, wie strichpunktiert dargestellt ist, von der Steuereinrichtung 5 entsprechend gesteuert werden. Beim kurzzeitigen Abschalten der Behandlungsstrahlung erfolgt eine Kontraktion des behandelten Gewebes am Augenhintergrund, welches ebenfalls eine Druckwelle bewirkt, die von den oben beschriebenen Detektoren (Detektoren der Fig. 4 bis 12) oder dem Interferrometer 19 (Fig. 2 und 3) erfasst werden können. Die Auswertung der so gewonnenen Messsignale erfolgt dann in der gleichen Weise, wie oben erläutert wurde.

### [Bezugszeichenliste]

- 1: Behandlungsstrahlquelle
- 2: Bestrahlungsoptik (z. B. Spaltlampe)
- 3: Zusätzliche Strahlquelle
- 4: Schalteinrichtung
- 5: Steuereinrichtung
- 6: Detektor (akustischer)
- 7: Detektor (optischer)
- 8: Auswerteeinrichtung
- 9: Transientenrecorder
- 10: Rechner
- 11: Temperatur-Anzeigeeinrichtung
- 12: Lichtleiter (Glasfaser)
- 13: Steuerelektronik für AO-Modulator
- 14: Schalter, z. B. akustooptischer Modulator
- 15: Koppleroptik
- 16: Auge
- 17: Kontaktglas
- 18: Akustisch absorbierende Schicht
- 19: Interferrometer
- 20: Messstrahlengang
- 21: Referenzstrahlengang
- 22: Photodiode
- 23: Referenzreflektor
- 24: Strahlquelle
- 25: Strahlteiler
- 26: Impedanzanpassung

## Patentansprüche

1. Vorrichtung zur Behandlung von biologischem Gewebe, das mit Laserstrahlung mit einer vorgegebenen Behandlungszeit behandelt wird, mit
- einer Laserstrahlquelle (1) zur Erzeugung einer gepulsten Behandlungsstrahlung,
- einer Steuereinrichtung (5), mit welcher während der vorgegebenen Behandlungszeit die Laserstrahlquelle (1) steuerbar ist,
- einer Bestrahlungsoptik (2), mit welcher die Behandlungsstrahlung auf das biologische Gewebe richtbar ist,
- einer zusätzlichen Laserstrahlquelle (3), welche während der Behandlungszeit zum Liefern zusätzlicher Strahlungspulse mit geringerer Energie und geringerer Pulsdauer als die Behandlungsstrahlung in die Bestrahlungsoptik (2) einkoppelbar ist,
- einem Detektor (6; 7; 19), mit welchem durch die zusätzliche Strahlung verursachte Expansionen und/oder Kontraktionen des Gewebes akustisch und/oder optisch messbar sind,
wobei
- an den Detektor (6; 7; 19) eine Auswerteeinrichtung (8) angeschlossen ist, welche eine die Proportionalität der durch die zusätzlichen Strahlungspulse verursachten Gewebeexpansionen und/oder -kontraktionen zur Gewebetemperatur angebende Eichkurve aufweist und das Messsignal des Detektors (6; 7; 19) in einen Temperaturwert während der jeweiligen Pulsdauer der Behandlungsstrahlung wandelt,
**dadurch gekennzeichnet, dass** das Messsignal des Detektors auf die Körpertemperatur des behandelten biologischen Gewebes normiert wird durch einen kurzzeitigen Strahlungspuls vor dem Einschalten der Behandlungsstrahlung.

2. Vorrichtung zur Behandlung von biologischem Gewebe, das mit einer Laserstrahlung mit einer vorgegebenen Behandlungszeit behandelt wird, mit
- einer Laserstrahlquelle (1) zur Erzeugung einer gepulsten Behandlungsstrahlung,
- einer Steuereinrichtung (5), mit welcher während der vorgegebenen Behandlungszeit die Laserstrahlquelle (1) steuerbar ist,
- einer Bestrahlungsoptik (2), mit welcher die Behandlungsstrahlung auf das biologische Gewebe richtbar ist,
- einem Detektor (6; 7; 19), mit welchem durch die Strahlung verursachte Expansionen und/oder Kontraktionen akustisch und/oder optisch messbar sind,
wobei
- eine zusätzliche Schalteinrichtung (4) vorgesehen ist, welche während der jeweils vorgegebenen Bestrahlungszeit die Behandlungsstrahlquelle (1) kurzzeitig aus- und wieder einschaltet,
- der Detektor (6; 7; 19) zur Erfassung der beim kurzzeitigen Abschalten und Wiedereinschalten der Behandlungsstrahlungsquelle (1) erzeugten Gewebeexpansion und/oder -kontraktion ausgebildet ist; und
- an den Detektor (6; 7; 19) eine Auswerteeinrichtung (8) angeschlossen ist, welche eine die Proportionalität der verursachten Gewebeexpansionen und -kontraktionen zur Gewebetemperatur angebenden Eichkurve aufweist und das Messsignaldes Detektors (6; 7; 19) in einen Temperaturwert während der jeweiligen Pulsdauer der Behandlungsstrahlung wandelt,
**dadurch gekennzeichnet, dass** das Messsignal des Detektors auf die Körpertemperatur des behandelten biologischen Gewebes normiert wird durch kurzzeitiges Ausschalten unmittelbar nach Einschalten der Behandlungsstrahlung.

3. Vorrichtung nach Anspruch 1 oder 2, **dadurch gekennzeichnet, dass** die Pulsdauer der zusätzlichen Strahlungspulse oder die Ausschaltzeit der Behandlungsstrahlung um einen Faktor 10² und einen höheren Faktor kleiner bemessen ist als die jeweiligePulsdauer der Behandlungsstrahlung.

4. Vorrichtung nach einem der Ansprüche 1 bis 3,
**dadurch gekennzeichnet, dass** die Pulsdauer der zusätzlichen Strahlungspulse oder die Ausschaltzeit der Behandlungsstrahlung im ns-Bereich liegt.

5. Vorrichtung nach Anspruch 1 oder 2,
**dadurch gekennzeichnet, dass** die Auswerteeinrichtung (8) an die Steuereinrichtung (5) angeschlossen ist.

6. Vorrichtung nach einem der Ansprüche 1 bis 5,
**dadurch gekennzeichnet, dass** die Auswerteeinrichtung (8) an eine Temperaturanzeigeeinrichtung (11) angeschlossen ist, welche die absoluten Temperaturwerte anzeigt.

7. Vorrichtung nach einem der Ansprüche 1 bis 6,
**dadurch gekennzeichnet dass** der Detektor (6; 7; 19) als akustisch oder optisch an das menschliche Augen koppelbarer Messwandler ausgebildet ist.

8. Vorrichtung nach Anspruch 7,
**dadurch gekennzeichnet, dass** der Detektor (6; 7) mittels eines auf das Auge aufsetzbaren Kontaktglases (17) an das behandelte Auge ankoppelbar ist.

## Claims

1. Apparatus for the treatment of biological tissue which is treated with laser radiation for a predetermined treatment time, comprising
- a laser beam source (1) for generating a pulsed treatment radiation,
- a control device (5) with which the laser beam source (1) is controllable during the predetermined treatment time,
- an optical irradiation means (2) with which the treatment radiation can be directed on to the biological tissue,
- an additional laser beam source (3) which can be coupled into the optical irradiation means (2) during the treatment time for delivering additional radiation pulses of lower energy and shorter pulse duration than the treatment radiation, and
- a detector (6; 7; 19) with which expansion and/or contraction of the tissue, caused by the additional radiation, can be acoustically and/or optically measured,
wherein
- connected to the detector (6; 7; 19) is an evaluation device (8) which has a calibration curve specifying the proportionality of the tissue expansion and/or contraction phenomena caused by the additional radiation pulses to the tissue temperature and which converts the measurement signal of the detector (6; 7; 19) into a temperature value during the respective pulse duration of the treatment radiation,
**characterised in that** the measurement signal of the detector is standardised to the body temperature of the treated biological tissue by a short-term radiation pulse prior to the treatment radiation being switched on.

2. Apparatus for the treatment of biological tissue which is treated with laser radiation for a predetermined treatment time, comprising
- a laser beam source (1) for generating a pulsed treatment radiation,
- a control device (5) with which the laser beam source (1) is controllable during the predetermined treatment time,
- an optical irradiation means (2) with which the treatment radiation can be directed on to the biological tissue, and
- a detector (6; 7; 19) with which expansion and/or contraction caused by the radiation can be acoustically and/or optically measured,
wherein
- there is provided an additional switching device (4) which switches the treatment beam source (1) off for a short time and on again during the respectively predetermined radiation time,
- the detector (6; 7; 19) is adapted to detect the tissue expansion and/or contraction produced when the treatment radiation source (1) is switched off for a short time and switched on again; and
- connected to the detector (6; 7; 19) is an evaluation device (8) which has a calibration curve specifying the proportionality of the tissue expansion and/or contraction phenomena caused to the tissue temperature and which converts the measurement signal of the detector (6; 7; 19) into a temperature value during the respective pulse duration of the treatment radiation,
**characterised in that** the measurement signal of the detector is standardised to the body temperature of the treated biological tissue by switching off for a short time immediately after the treatment radiation is switched on.

3. Apparatus according to claim 1 or claim 2 **characterised in that** the pulse duration of the additional radiation pulses or the switch-off time of the treatment radiation is shorter by a factor of 10² and a higher factor than the respective pulse duration of the treatment radiation.

4. Apparatus according to one of claims 1 to 3 **characterised in that** the pulse duration of the additional radiation pulses or the switch-off time of the treatment radiation is in the ns range.

5. Apparatus according to claim 1 or claim 2 **characterised in that** the evaluation device (8) is connected to the control device (5).

6. Apparatus according to one of claims 1 to 5 **characterised in that** the evaluation device (8) is connected to a temperature display device (11) which displays the absolute temperature values.

7. Apparatus according to one of claims 1 to 6 **characterised in that** the detector (6; 7; 19) is in the form of a measuring transducer which can be acoustically or optically coupled to the human eye.

8. Apparatus according to claim 7 **characterised in that** the detector (6; 7) can be coupled to the treated eye by means of a contact lens (17) which can be fitted on to the eye.

## Revendications

1. Dispositif de traitement de tissu biologique qui est traité par du rayonnement laser avec une durée de traitement prescrite, comprenant
- une source ( 1 ) de rayon laser pour produire un rayonnement de traitement pulsé,
- un dispositif ( 5 ) de commande, par lequel la source ( 1 ) de rayon laser peut être commandée pendant la durée de traitement prescrite,
- une optique ( 2 ) de rayonnement, par laquelle le rayonnement de traitement peut être dirigé sur le tissu biologique,
- une source ( 3 ) supplémentaire de rayon laser qui peut être accouplée à l'optique ( 2 ) de rayonnement pendant la durée de traitement pour fournir des impulsions de rayonnement supplémentaires d'une énergie plus petite et d'une durée d'impulsion plus petite que celles du rayonnement de traitement,
- un détecteur ( 6 ; 7 ; 19 ) par lequel les dilatations et/ou les contractions du tissu provoquées par le rayonnement supplémentaire peuvent être mesurées acoustiquement et/ou optiquement,
dans lequel
- il est raccordé au détecteur ( 6 ; 7 ; 19 ) un dispositif ( 8 ) d'exploitation qui a une courbe d'étalonnage indiquant la proportionnalité à la température du tissu des dilatations et/ou contractions du tissu provoquées par les impulsions de rayonnement supplémentaires, et le signal de mesure du détecteur ( 6 ; 7 ; 19 ) est transformé en une valeur de température pendant chaque durée d'impulsion du rayonnement de traitement,
**caractérisé en ce que** le signal de mesure du détecteur est normé sur la température du corps du tissu biologique traité par une impulsion de rayonnement de courte durée avant le branchement du rayonnement de traitement.

2. Dispositif de traitement de tissu biologique qui est traité par du rayonnement laser avec une durée de traitement prescrite comprenant
- une source ( 1 ) de rayon laser pour produire un rayonnement de traitement pulsé,
- un dispositif ( 5 ) de commande, par lequel la source ( 1 ) de rayon laser peut être commandée pendant la durée de traitement prescrite,
- une optique ( 2 ) de rayonnement, par laquelle le rayonnement de traitement peut être dirigé sur le tissu biologique,
- une source ( 3 ) supplémentaire de rayon laser, qui peut être accouplée à l'optique ( 2 ) de rayonnement pendant la durée de traitement pour fournir des impulsions de rayonnement supplémentaires d'une énergie plus petite et d'une durée d'impulsion plus petite que celles du rayonnement de traitement,
dans lequel
- il est prévu un dispositif ( 4 ) supplémentaire de commutation qui, pendant la durée de rayonnement respectivement prescrite, branche et redébranche brièvement la source ( 1 ) de rayon de traitement,
- le détecteur ( 6 ; 7 ; 19 ) est constitué pour détecter la dilatation et/ou la contraction du tissu produites lors du débranchement de courte durée et du rebranchement de la source ( 1 ) de rayonnement de traitement ; et
- il est raccordé au détecteur ( 6 ; 7 ; 19 ) un dispositif ( 8 ) d'exploitation qui a une courbe d'étalonnage indiquant la proportionnalité à la température du tissu des dilatations et/ou contractions du tissu provoquées par les impulsions de rayonnement supplémentaires, et le signal de mesure du détecteur ( 6 ; 7 ; 19 ) est transformé en une valeur de température pendant chaque durée d'impulsion du rayonnement de traitement,
**caractérisé en ce que** le signal de mesure du détecteur est normé sur la température du corps du tissu biologique traité par des branchements de courte durée immédiatement après le branchement du rayonnement de traitement.

3. Dispositif suivant la revendication 1 ou 2, **caractérisé en ce que** la durée des impulsions de rayonnement supplémentaires ou la durée de débranchement du rayonnement de traitement est plus petite d'un facteur de 10² et d'un facteur plus grand que la durée d'impulsion respective du rayonnement de traitement.

4. Dispositif suivant l'une des revendications 1 à 3,
**caractérisé en ce que** la durée des impulsions de rayonnement supplémentaires ou la durée de débranchement du rayonnement de traitement est de l'ordre de la ns.

5. Dispositif suivant la revendication 1 ou 2,
**caractérisé en ce que** le dispositif ( 8 ) d'exploitation est raccordé au dispositif ( 5 ) de commande.

6. Dispositif suivant l'une des revendications 1 à 5,
**caractérisé en ce que** le dispositif ( 8 ) d'exploitation est raccordé à un dispositif ( 11 ) d'indication de la température qui indique des valeurs de température absolue.

7. Dispositif suivant l'une des revendications 1 à 6,
**caractérisé en ce que** le détecteur ( 6 ; 7 ; 19 ) est constitué sous la forme d'un convertisseur de mesure pouvant être accouplé acoustiquement ou optiquement à l'oeil humain.

8. Dispositif suivant la revendication 7,
**caractérisé en ce que** le détecteur ( 6 ; 7 ) peut être accouplé à l'oeil traité au moyen d'un verre ( 17 ) de contact pouvant être mis sur l'oeil.
